# EUROPEAN PATENT APPLICATION

(11) **EP 1 538 537 A2**
(43) Date of publication of application: **08.06.2005**
(21) Application number: 04028319.4
(22) Date of filing: 30.11.2004
(51) Int. Cl.: G06F 17/30

(54) **Method of storing fast throughput experimentation information in a database**

(30) Priority: 01.12.2003 US 525970 P
(71) Applicant: Accelrys Software, Inc., San Diego California 92121 (US)
(72) Inventor: Fitzgerald, George, Carlsbad CA 92009 (US); Hill, Jörg-Rüdiger, 82041 Deisenhofen (DE); Tucker, Joseph B., Cambridge CB4 3NG (GB)
(74) Representative: Sternagel, Fleischer, Godemeyer & Partner Patentanwälte

(57) **Abstract**

A database is disclosed for storing fast throughput experimentation information. The database is organized in a hierarchical structure such that various database objects have a parent-child relationship. The hierarchical structure facilitates control over user access to various parts of the database. A method is disclosed for automatically populating the database with sample definitions. The sample information is provided in an arbitrary electronic format, which is imported into the database. A method is also disclosed for tracking sample events. Whenever a user takes an action in the database that affects a sample, a sample event is recorded. Finally, a method for automatically importing experimental data resulting from fast throughput experimentation on samples is disclosed. The data is automatically associated with information already stored in the database, such as the test conditions that held during the experimentation and the sample recipes of the samples tested.

## Description

The work described herein was partially funded by the United States Department of Energy under DOE Project DE-FC26-02NT41218, "Discovery of new NOx reduction catalysts for CIDI engines using combinatorial techniques."

### Related Application

This application claims priority to U.S. Provisional Application No. 60/525,970, entitled "Method of Storing Fast Throughput Experimentation Information in a Database," filed on December 1, 2003, which is incorporated herein by reference in its entirety.

### Background of the Invention

### Field of the Invention

The present invention relates to databases used in fast throughput experimentation. More particularly, the present invention relates to methods and systems for populating such a database with sample information, test procedures, and test results and for tracking sample information.

### Description of the Related Art

Fast throughput experimentation (FTE) is now widely applied to identify promising new compounds. For example, combinatorial chemistry can be used to accelerate the rate of pharmaceutical discovery. FTE can also be used in material science synthesis to identify catalysts, polymers, and other useful materials. Furthermore, FTE is useful in material science formulation, aiding in the development and improvement of paints, coatings, drugs, detergents, foods, cosmetics, petrochemicals, and other formula-based materials through the combination and processing of existing ingredients. FTE allows hundreds of experiments to be conducted in the time it formerly took to complete just one.

In FTE, a large number of samples are defined and created in a fast-throughput manner. The definition of each sample includes its composition and the processing operations that are applied to it. For example, the composition can include the ingredients added to each sample and the amount of each ingredient. Processing operations can include steps such as the order ingredients are added, mixing, heating, reaction times, etc. Typically, a family or library of samples is created consisting of a set of samples with the same processing operations applied to a set of systematically varying ingredients. In other libraries the processing operations are varied systematically as well. Once the samples are defined, they can be created in a laboratory.

In some cases, synthetic procedures and formulation recipes used in FTE are stored in paper filing systems, notebooks, or on individual personal computers. However, such a storage system does not ensure that all personnel involved in a project works with, and contributes to, a consistent data resource. Furthermore, the use of a decentralized storage system may result in experimenters needlessly creating samples that have already been created.

Once samples have been created, they are usually tested to characterize the samples or to screen the samples for particular properties. Testing is advantageously conducted in a fast-throughput manner. In order to assure repeatability of the experiments, it is necessary to record as much information as possible about the experimental conditions and the way tests were performed. Normally, the individual conducting the experiment will have an understanding of the important conditions, and he or she will record this in a laboratory notebook. However, use of such individual records may result in experimenters needlessly repeating tests. In addition, details regarding tests performed, the actual test results, and detailed sample recipe information may not be located in the same conveniently accessible location.

Due to the large number of samples created and tests performed, tracking the history (e.g., experiments and processing performed on a sample) and the location of samples can also be a challenge. Tracking sample history and location is especially important in materials science. In areas such as fast-throughput drug discovery or heterogeneous catalysis, samples are usually synthesized, tested once, and discarded. In materials science, however, synthesis may be considerably more costly in terms of time and resources. Samples, therefore, might be subjected to several tests before being discarded.

When samples are not immediately discarded after creation, they may be moved from one location to another, typically from storage to an experimental apparatus, and back to storage. In such cases, it is necessary to track the location of samples in order to use them in subsequent experiments. Samples may also be routinely subjected to post-synthesis treatments (e.g., processing steps applied after the samples have been created). It is essential to know which treatments have been applied to which samples because subsequent processing may alter the characteristics of a sample. It is also important to track experiments performed on a sample. The results obtained for a sample in one experiment may depend on the conditions to which it was exposed in earlier experiments. In order to ensure repeatability of experimental results, it is critical to know all the earlier experiments that each sample was subjected to.

Thus, there exists an unmet need in the art for a centralized database in which to store information regarding FTE sample definitions, the conditions of tests performed on the samples, the data resulting from the tests performed, and sample tracking information. Furthermore, because entering such information into a central database can be tedious and time consuming, there exists an unmet need for a method of quickly and conveniently populating the database with sample definitions, sample test results, and sample tracking information.

### Summary of the Invention

One aspect of the present invention is a method of searching a database, the database comprising a plurality of sample data objects, each sample data object corresponding to a sample that comprises a chemical or composition of chemicals and each sample data object comprising a plurality of data fields populated with sample events, the method comprising: 1) constructing a query to search for one or more of the sample data objects based on information contained in one or more of the data fields; and 2) performing a search of the database using the query. In one embodiment, a graphical user interface is provided that is adapted to receive an input data especially from a user and use the input data to construct a query according to this method.

Another aspect of the present invention is a method of identifying the location of one or more samples, each sample comprising a chemical or a composition of chemicals, the method comprising: 1) providing a database comprising sample data objects corresponding to the samples, each of the sample data objects comprising a plurality of data fields populated with sample events and locations of the samples when the sample events occur; 2) retrieving locations from the database corresponding to the sample event recorded last for at least one of the samples; and 3) populating cells in a spreadsheet with the locations corresponding to the sample event recorded last. In one embodiment, a graphical user interface is provided that is adapted to receive an input data especially from a user and use the input data to identify samples and adapted to determine the location of the samples using this method.

Another aspect of the present invention is a method of populating a database with experimental results obtained from conducting an experiment on a plurality of samples, the method comprising: 1) providing a database stored on a machine readable medium, the database comprising a plurality of sample data objects, each sample data object corresponding to a sample comprising a chemical or a composition of chemicals; 2) creating a test data object corresponding to an experiment that can be conducted on the samples; 3) defining in the test data object information regarding format of data obtained upon conducting the experiment; 4) selecting a data file using a Common File Dialog program, e.g. Windows® Common File Dialog, the data file comprising experimental results obtained from conducting the experiment on the samples; and 5) using the format, reading the data file and populating data fields with the experimental results for each sample.

Another aspect of the present invention is a method of populating a database with information pertaining to a plurality of samples, each sample comprising a chemical or composition of chemicals, the method comprising: 1) providing in an arbitrary electronic format information corresponding to ingredients and/or process steps used to synthesize each sample; 2) reading the information in arbitrary electronic format; and 3) populating data fields in the database with the ingredients and process steps for each sample. In one embodiment, a graphical user interface is provided that is adapted to receive input data especially from a user and use the input data to provide the above information and the interface is adapted to then perform the reading and populating steps.

### Brief Description of the Drawings

FIGURE 1 depicts a hierarchical display for accessing database objects.

FIGURE 2 depicts a display for viewing and accessing database objects of the analytical equipment type.

FIGURE 3 depicts a detailed database object window for viewing and editing a project database object.

FIGURE 4 depicts a detailed database object window for viewing and editing a study database object.

FIGURE 5 depicts a detailed database object window for viewing and editing a library database object.

FIGURE 6 depicts a detailed database object window for viewing and editing a sample database object.

FIGURE 7 depicts a graphical display of a plate with samples assigned to it.

FIGURE 8 depicts a detailed database object window for viewing and editing an experiment database object containing a hierarchical experimental-results child list.

FIGURE 9 depicts a hierarchical structure of a fast throughput experimentation database.

FIGURE 10 depicts a history of sample events.

FIGURE 11 is a flowchart illustrating the automatically populating a database with samples.

FIGURE 12 depicts a window to facilitate the creation of a library of samples using a library design tool.

FIGURE 13 depicts a window for entering value ranges of variable parameters in a library design tool.

FIGURE 14 depicts a spreadsheet containing sample information to be automatically imported into a database.

FIGURE 15 is a flowchart illustrating the use of collections containing subsets of sample information to facilitate populating a database with sample information.

FIGURE 16 is a flowchart illustrating copying existing sample information to facilitate populating a database with sample information.

FIGURE 17 is a flowchart illustrating the automatic importation of experimental results into a database.

### Detailed Description of the Preferred Embodiment

One embodiment of the present invention provides a computer-implemented FTE database organized into a hierarchical structure. The system provides a graphical user interface to a database that enables a user to view, navigate to, and modify database objects contained within the hierarchical structure of the database. Database objects are created and associated with each other in a hierarchical manner such that various objects have a parent-child relationship. In some cases, objects can have multiple parent and children objects. Some objects may have no parent and/or child objects. Each database object can contain fields for holding information pertaining to the object. Generally, each database object includes fields for the name and description of the object and the identity of the user who created the object. Some database objects can also contain a field that identifies the parent object to which the object belongs. In addition, database objects can contain one or more child lists. The child lists contain objects at lower levels in the hierarchy (child objects) that help define the present object, objects that are in some other way associated with the present object, or a description or properties of the object. Some child lists can automatically be populated based on associations indicated in other object definitions. For example, an object that references another object as its parent can automatically be included in a list of child objects contained within one of the parent's child lists. Other child lists can be manually populated, e.g. by a user, or automatically by a populating apparatus. Not all database objects contain child lists. Furthermore, while the above-described format for database objects is advantageous, database objects can have any format.

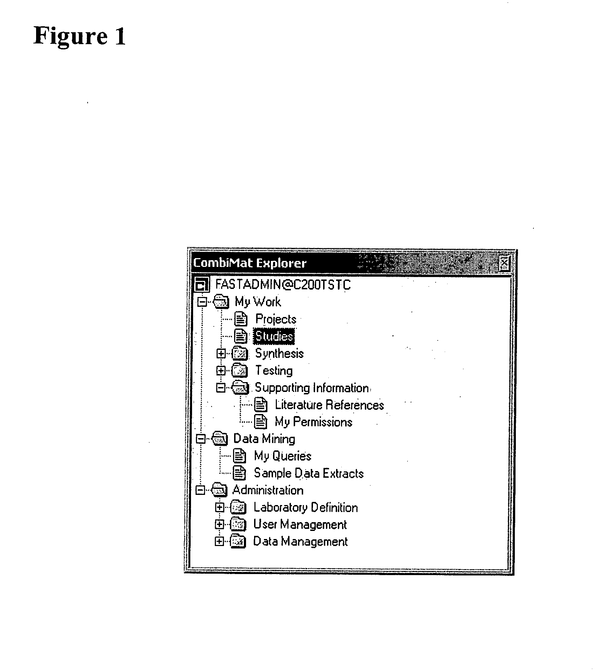

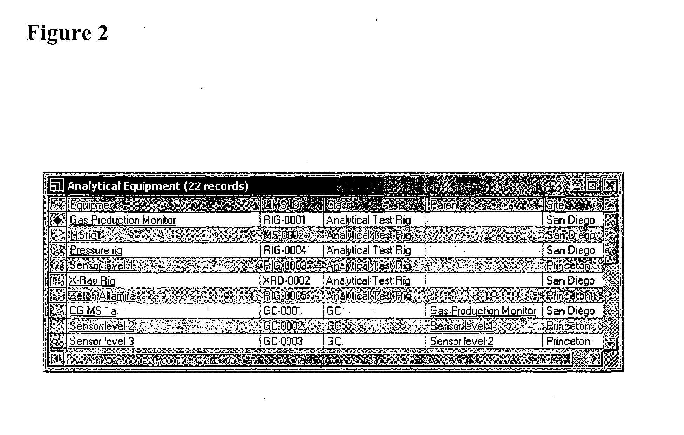

In one embodiment, a graphical display is provided that represents all of the available types of database objects in a hierarchical tree structure, such as depicted in Figure 1. Folders and lists are provided such that a user may expand each folder to reveal lists of subfolders and database object types that are contained within each folder. If a user selects a displayed database object type, a list of all of the database objects of the indicated type that have been created is displayed. For example, if a user selects the database object type "Analytical Equipment," a list of all analytical equipment database objects that have been created is displayed, such as depicted in Figure 2. Selecting an individual database object from the list of created objects allows viewing, and in some cases, editing of the object provided that the user has permission to view and/or edit the object. Alternatively, a new database object of the selected type may be created.

In one embodiment, once a particular database object is selected or the user indicates that he wishes to create a new database object, a graphical window is displayed that provides one or more fields in which a user can view and enter information to define the object. In some cases, information in the fields will automatically be supplied. Also accessible from the graphical window are the one or more child lists of objects. Selecting objects within a child list allows viewing, and in some cases, editing of the object via a similar graphical window, provided that the user has permission to view and/or edit the object. Alternatively, the user may populate some child lists with new database objects.

Examples of database object types that can be created include, but are not limited to, projects, studies, libraries, samples, process steps, chemical ingredients, equipment, parameters, units, users, locations, plates, plate types, tests, and experiments. Following is a description of each of these objects. It is to be understood that these database objects are merely provided as examples and that their existence is not required. Furthermore, the descriptions of the information contained within each database object and their format is purely illustrative. Any information and format may be used.

### Examples of Database Obiects

### Projects

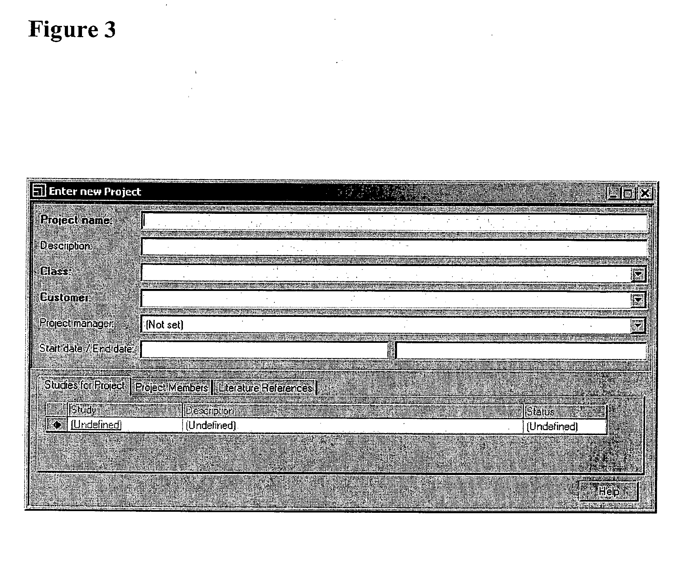

Projects are generally the highest level in the database hierarchy in that projects have no parent objects. Project database objects typically identify a set of defined goals in a research and development program and specify start and end dates. An example of a project goal is to identify catalysts that remove nitrogen oxides from an exhaust stream. Projects can contain child lists identifying the studies within the project and users who are members of the project. Figure 3 represents a graphical window allowing a user to enter the project information and view the child lists associated with the project.

### Studies

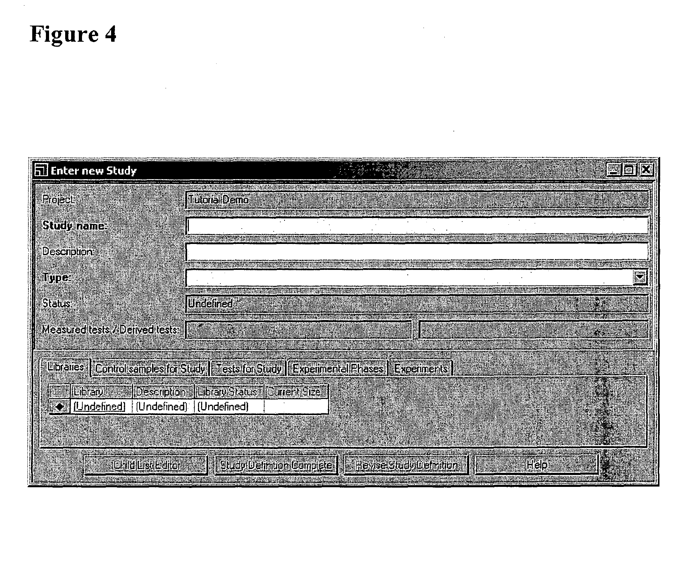

Once a project has been created, the studies child list within the project may be populated with studies. Studies are generally used to segment the work of a project into categories. An example of a study within a nitrogen-oxide catalyst project is to synthesize and test zeolite based catalysts. Another example is to synthesize and test metal oxide based catalysts. A graphical window facilitating the creation of a study is illustrated in Figure 4. Each study definition contains a reference to the project in which it belongs. Because the study is being created as part of a child list for the project, the project field can automatically be completed. Studies can contain child lists indicating the libraries contained within each study, the tests that are used as part of the study, and the experiments that are conducted during the study. A user can populate the test child list with pre-defined test database objects. As described below, when the user populates the test child list, they can specify the value of any parameters associated with the tests.

### Libraries

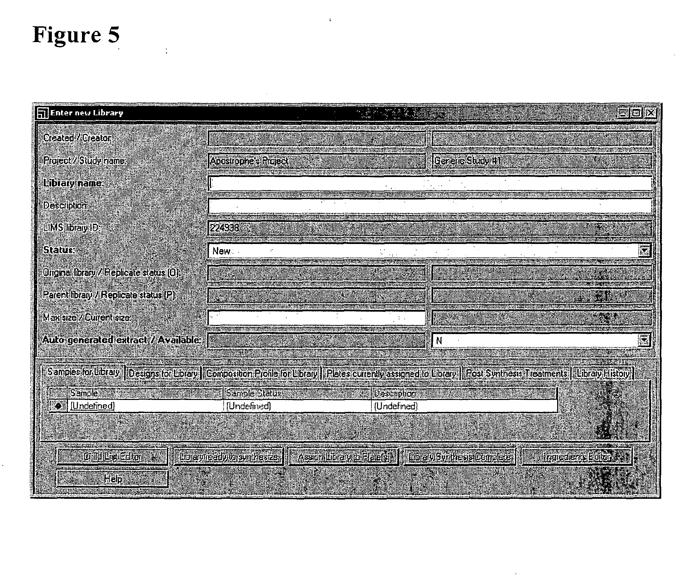

Libraries are used to group sets of samples that are usually related in some way. For example, the samples may contain the same ingredients in varying concentrations. However, even an unrelated set of samples may constitute a library. An example of a library within a metal oxide based catalyst study is a set of samples containing silver, zinc, and cerium in varying amounts. Another example is a set of samples containing silver, cobalt, platinum, and zinc. Library database objects can be created by populating the library child list within a study definition. Library definitions include a field for the study in which they belong. This field can automatically be completed. Libraries can contain child lists for the samples that make up the library, plates to which samples within the library have been assigned, post-synthesis treatments that are applied to all of the samples within the library, and a history of the library. A user and/or a device may populate the post-synthesis treatment child list with process steps that are applied to every sample within a library after the samples have been synthesized. The order of the process steps in this child list represents the order that the process steps are to be applied to the samples. Figure 5 depicts a graphical window allowing a user to enter the library information and view the child lists associated with the library.

In one embodiment, new libraries are created by copying an existing library in order to avoid a user having to reenter information. The copy of a library may be put in the same study as the existing library or in a different study. The existing library is unaltered by the copying process. Once a copy is made, the new library can be modified. The copying of libraries can be tracked by including replication status fields within the library definitions. For example, an original-library-replicate-status field can indicate whether or not the library is an exact replicate of the original library. This indicator applies even if the copy is several times removed from the original (e.g., a copy of a copy). A parent-library-replicate-status field can indicate whether or not the library is an exact replicate of its immediate predecessor (i.e., the library from which it was actually copied). The replicate status fields can automatically be completed according to whether copies are modified or not.

### Samples

Sample database objects can be created by populating the sample child list within a library. The sample definition includes a field for the library to which the sample belongs. This field can automatically be completed. In one embodiment, a field for the status of a sample is also provided. Status indicators that can be used include, but are not limited to, new, ready to synthesize, synthesis in progress, synthesized, cannot be synthesized, synthesis failed, disposed of, and deleted. A new status indicates that the definition of the sample database object is in progress. A ready-to-synthesize status indicates that the sample database object definition is complete but the sample has not yet been synthesized in the laboratory. A synthesis-in-progress status indicates that the sample is in the process of being synthesized in the laboratory. A synthesized status indicates that sample synthesis is complete. A cannot-be-synthesized status indicates that it is not possible to actually synthesize the sample in the laboratory. A synthesis-failed status indicates that synthesis was attempted in the laboratory but it failed. A disposed-of status indicates that the physical sample was destroyed but the database object remains. Finally, a deleted status deletes the sample object from the database.

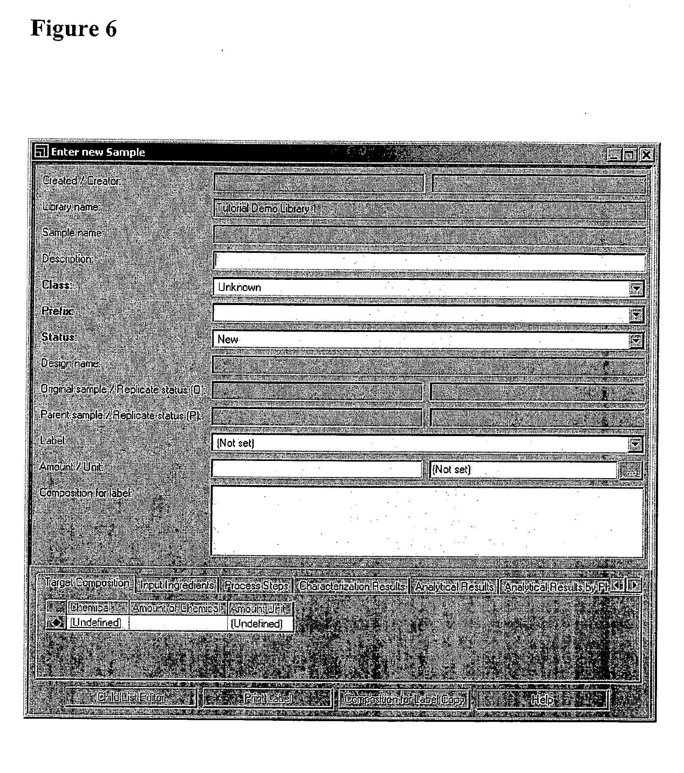

Child lists within a sample database object can include the ingredients that make up the sample, process steps used to create the sample, the experimental results from tests performed on the sample, and the history of the sample. Figure 6 represents a graphical window allowing a user to enter the sample information and view the child lists associated with the sample.

The user and/or a device can populate the child list of process steps by selecting from process steps that have been pre-defined. Process steps are the experimental steps that need to be carried out in order to synthesize a sample. Examples are adding an ingredient, heating, mixing, drying, and the like. The order of the process steps in the process-step child list indicates the order that the steps are to be carried out in the laboratory when synthesizing the sample. When a user selects a process step for inclusion in a sample's child list, he can be given the opportunity to specify the value of any parameters that have been included in the process step definition. For example, the user may select a process step called "Add Ingredient." The "Add Ingredient" process step may have an ingredient parameter and an amount parameter associated with it. The user can then select which ingredient is to be added during the process step by selecting from a list of pre-defined ingredients and indicating a value for the amount of ingredient that is to be added. The child list of ingredients in a sample database object can automatically be updated when a process step for adding an ingredient is included.

As in the case for libraries, samples can be copied either into the same library or into different libraries to avoid repetitious entering of information. The copied samples can be modified as desired. Also as in the case for libraries, fields can be provided to track sample copying. For example, an original-sample-replicate-status field can indicate whether or not the sample is an exact replicate of the original sample. This indicator applies even if the copy is several times removed from the original (e.g., a copy of a copy). A parent-sample-replicate-status field can indicate whether or not the sample is an exact replicate of its immediate predecessor (i.e., the sample from which it was actually copied). The replicate status indicator fields can automatically be completed by detecting whether copies are modified or not. Further tracking of replicates can be provided by a child list indicating all of the samples that are replicates of the current sample.

### Chemical Ingredients

Chemical ingredients can be independently defined prior to their inclusion in the definition of a sample. The definition of chemical ingredients need not contain reference to a particular parent object. For example, a defined chemical ingredient is likely to be a constituent in more than one sample. Possible child lists contained within a chemical ingredient definition include the libraries that contain the ingredient and other chemical ingredients that are constituents of the ingredient. The child lists for the libraries that contain the chemical ingredient can be automatically updated whenever adding the ingredient is part of a process step or a sample within a library.

### Process Steps

Like chemical ingredients, process steps can be defined independent of particular parent objects. However, process steps can themselves be arranged in a hierarchical structure such that a particular process step can consist of a series of other process steps. For example, a process step can consist of the three procedures: two for adding two ingredients and one for mixing the two ingredients. As such, a process step definition can contain a field for reference to a parent process step to which the process step belongs as a child. Similarly, a process step can contain a child list indicating the child process steps that make up the present process step. Process steps can also contain a child list of parameters that are required for the process step. For example, a process step for heating may require that a parameter be defined indicating the temperature to heat to.

A user can populate the lists of child processes and parameters by selecting from pre-defined processes and parameters or he can create the child processes and parameters at the same time that he populates the lists. The order of child processes within the process list indicates the order that the processes are to be conducted in the laboratory. Parameters

Process step and experimental test database objects can require specification of one or more parameters as part of their definition. Generally, parameters are laboratory variables such as concentration, temperature, and pressure. However, any type of parameter that needs to be associated with a process step or experimental test can be created. The definition of a parameter can include whether it is to be numeric only, what unit the parameter has if the unit is to be fixed (e.g., degrees Celsius), or what family of units is associated with the parameter (e.g., temperature). When a family of units is selected, the user is allowed to select a specific unit within the family when specifying the value of the parameter. Typically, parameters have no child lists. The value that particular parameters are to have are set at the time of sample definition in the case of parameters associated with process steps and at the time of populating the test child list in a study database object in the case of parameters associated with tests.

### Units

Unit database objects can be used for association with parameters or data. Both unit families and specific units can be defined. As discussed above, unit families are unit types such as temperature. An example of a specific unit within the temperature unit family is degrees Celsius. When specific units are defined, the unit family to which the unit belongs can be identified as part of the definition. A unit can also be identified as being the standard unit within a unit family. The non-standard units can be assigned offset or factor values that indicate how to convert the non-standard unit into the standard one. In this way, after parameter values having a specified unit have been set, a computer-implemented method can convert the parameter value from one unit into another unit within the same family.

### Equipment

The equipment database object contains information regarding laboratory equipment used to synthesize samples or perform analytical tests on existing samples. Equipment may be organized into a hierarchical structure such that a parent piece of equipment (e.g. a "test rig") can contain multiple pieces of child equipment (e.g., "sensors"). Multiple levels can be created in the hierarchy. In the case of child equipment, the parent equipment can be identified as part of the child's definition. Parent equipment can contain a child list consisting of all child equipment that resides on the parent equipment. Other information such as site location of the equipment, the serial number, equipment manufacturer, purchase date, and purchase price can be included in the equipment definition. Other child lists can include the tests that the equipment can perform and the history of the piece of equipment.

### Tests

Tests for characterizing and analyzing samples can be defined as database objects. Characterization tests are used to determine the properties of a sample. Analytical tests are generally used to screen samples with respect to target properties. Examples of tests include measuring X-ray diffraction, catalytic activity, superconductivity, tensile strength, temperature, mass spectrum, flow rate, or infrared spectrum. Each test may be associated with a piece of equipment such that equipment and tests can have a parent-child relationship. However, each piece of equipment can have multiple tests associated with it. A user can create tests by populating the test child list contained in the parent equipment definition. The equipment that a particular test uses is automatically supplied as part of the test definition. Generally, tests are created for equipment at the lowest level in the equipment hierarchy (e.g., "sensors").

Test definitions can contain the format of the data that results from performing the test. For example, the user can specify whether data associated with the test will include numerical data or textual data. Furthermore, the user can specify whether the data consists of single data points, conjoined points such as pairs of results (e.g., x, y), triplets of results (e.g., x, y, z), and so on up to arbitrary order. Additionally, it can be specified that the data can consist of an array of any of these data types. As an example, a test to measure an infrared spectrum would consist of an array of (x, y) pairs of points where one value of each pair represents a frequency and the second value represents the intensity of absorption. The data format included in a test definition can be used to facilitate importing data, searching data, and displaying data.

After the format of the test data has been specified, the units associated with each piece of data can be defined. For example, for an infrared spectrum, a user can specify that the x value has the unit of inverse centimeters and the y value has the unit of transmittance. The user can select the appropriate unit from a list of pre-defined units.

Test database objects can also contain a child list of parameters that a user wishes to associate with a particular test. For example, a user may wish to specify that the test is to be conducted at pH 7.2. In that case, the user can populate the parameter child list by selecting a parameter entitled "pH" from a list of pre-defined parameters. The value of 7.2 can be assigned when the test is assigned to the test child list in the study database object that contains the samples to be tested. In some cases, a range of values can be assigned to a parameter.

### Plate Types

The plate type database object contains information regarding the physical format of plates that are to be used in analytical tests. Generally, plates used in the laboratory contain multiple wells into which samples are placed for FTE. For example, a plate may contain 96 wells. The plate type database object includes values for the number of rows and columns of wells the plate contains. The layout of the wells can also be defined. For example, the wells may be in a hexagonal layout. In some cases, a plate may contain a single well.

### Plates

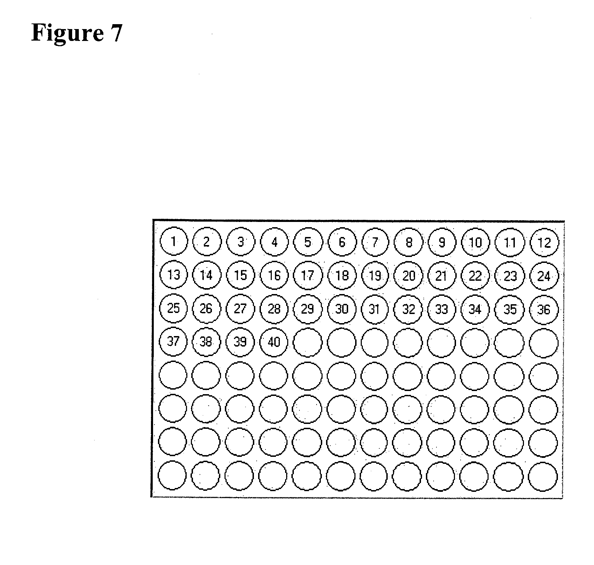

A plate database object is a specific instance of a particular plate type containing specific samples. A user may define a plate by specifying the plate type and then assigning samples to the plate. The samples that are assigned to a plate are indicated in a child list. The child list also indicates which well each sample is assigned to, such as by identifying the well number. In one embodiment, a graphical display depicting the plate is provided to facilitate a user assigning samples to the plate and viewing sample locations on the plate. The selected plate type determines the number and location of wells depicted. Figure 7 shows a graphical display depicting a standard 96-well rectangular plate type with 40 samples assigned to it. The wells in the plate can be populated automatically by sequentially selecting samples from lists of pre-defined samples. Multiple samples can also be selected simultaneously and the wells populated automatically. Alternatively, a user can identify the specific well to which a sample is to be assigned.

Possible additional child lists that can be included in a plate definition include post-synthesis treatments that are applied to the plate and a list of all the events that have affected a plate (i.e., the plate history). A user can populate the post-synthesis treatment child list with process steps that are to be applied to every sample assigned to the plate. The order of the process steps in the child list represents the order that the process steps are applied to the samples.

The plate definition can also include the status of a plate and the physical location of the plate. Examples of plate status include, but are not limited to, registered, samples assigned, samples on plate, in transit, recycled, and deleted. A registered status indicates that the plate has been defined but no samples have been assigned to it. A samples-assigned status indicates that samples have been assigned to the plate in the database but the samples have not yet been physically placed in a plate in the laboratory. A samples-on-plate status indicates that the samples have physically been placed on a plate in the laboratory. An in-transit status means that a physical plate containing samples is in the process of moving from one location to another (e.g., from storage to the laboratory or from one site to another). Recycled status indicates that a plate has been physically destroyed but the record remains in the database. Deleted status removes the plate record from the database.

### Experiments

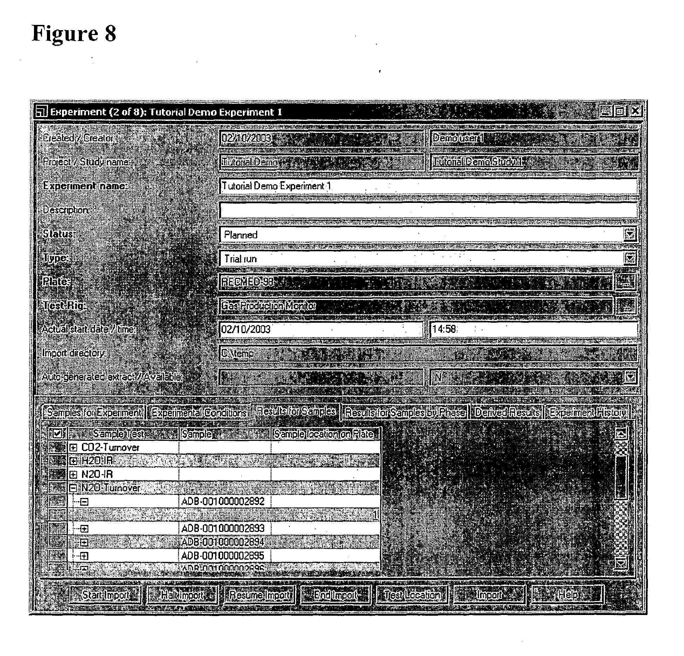

In general, an experiment database object assigns analytical equipment apparatus to a plate containing samples. As such, an experiment definition contains reference to an equipment database object and a plate database object. The experiment database object indicates that tests that are associated with the equipment and any associated child equipment are to be conducted on the samples on the plate. Only the tests that have been added to the test child list in the study database object that contains the samples can be conducted. The experiment database object can also contain child lists that indicate the samples that are contained on the plate and the experimental results. The experimental-results child list can be displayed in a hierarchical tree structure with the tests that are part of the experiment at a higher level in the hierarchy and each sample tested for each test at a lower level as illustrated in Figure 8. Each sample under each test may be selected to view the experimental data that resulted from conducting the test on the sample in the laboratory. The experimental-results child lists can automatically be populated by importing the data resulting from actual laboratory testing as described below. When the experimental-results child list is populated with test results, the experimental-results child lists located in the sample database object of each sample tested are also automatically populated.

### Locations

Location database objects can be used to locate samples, plates, equipment, or any other physical object represented in the database when they are associated with those objects. Location database objects can be organized in a hierarchical structure. For example, a parent location may be a particular facility. That parent location may have as a child a particular laboratory within the parent facility. Furthermore, the child location may also have a child that identifies a particular rack within the laboratory. Location database objects contain the name and description of the location and a reference to parent locations. Furthermore, location database objects can contain a child list indicating any child locations.

Database objects representing items whose location needs to be tracked can include a field for the item's location. When defining the database object, a user can populate the location field by selecting from a list of pre-defined locations. Furthermore, whenever the physical location of the item changes, a user can modify the database object's location field as appropriate.

### Users

A database administrator may define users of the database. User database objects contain identity and login information. Furthermore, user definitions can contain child lists that indicate the database objects that the user has permission to access. Permissions can be granted independently for viewing, creating, and/or modifying the objects. The hierarchical structure of the database interface can facilitate control over database access. For example, users who are granted access to a project can have access to all database objects associated with that project that are at lower levels in the hierarchy (e.g., studies, libraries, and samples). Alternatively, users granted access to lower levels in the hierarchy (e.g., studies or libraries) can have access to database objects even lower in the hierarchy (e.g., samples) but not objects higher in the hierarchy (e.g., projects). In addition, certain database objects may be restricted such that only an administrator can create and modify them. For example, it may be advantageous to restrict creation and modification of database objects that do not need to be created or modified often, such as equipment, parameters, units, plate types, and users.

User database objects can also be referenced in any other database objects to indicate the creator of that database object. For example, creator fields within other database objects can be automatically populated when a logged-in user creates that object. In one embodiment, permission for database access can be based on whether a user is a member of a project or the creator of a particular object. For example, an administrator can specify that all users who are members of a particular project can create studies and libraries within the project, but only the creator of a library has permission to modify it.

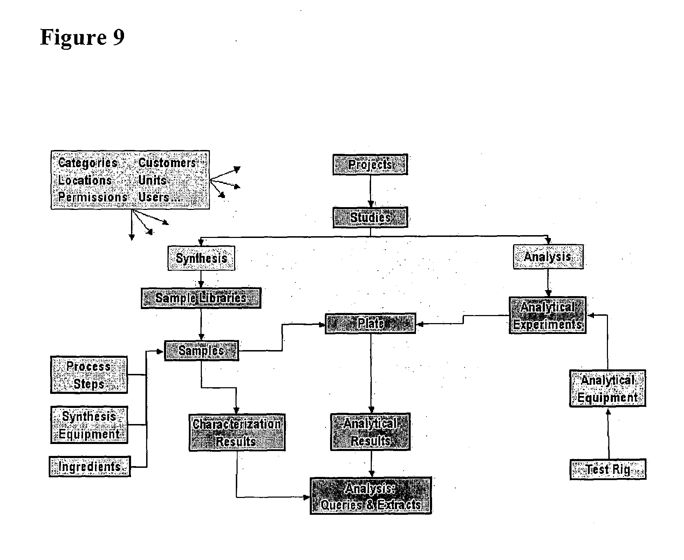

Figure 9 depicts the hierarchical structure of some of the above-described database objects. As illustrated, certain database objects, such as locations, units, and users can be associated with many of the other database objects at any level in the hierarchy. Other objects, such as projects, studies, and libraries have strict parent-child relationships. The database objects described above and the structure depicted in Figure 9 represent one example of a hierarchical FTE database interface and is not intended to be limiting. Any type and format of FTE database object may be used. Furthermore, any hierarchical structure can be created.

### Tracking the Sample History

As indicated above, sample database objects can contain a sample-history child list. The sample-history child list contains a list of all of the events that have affected the sample in some way. The sample-history child list can also contain the date that the event occurred. As used herein, a "sample event" refers to any action taken with respect to a sample in the database. Whenever such an action takes place, one or more sample events are recorded. For example, whenever a field or another child-list contained within a sample database object is altered, the sample-history child list can be updated to reflect the change. In other cases, a sample event may occur when actions are taken with respect to other database objects that are related to the sample.

In one embodiment, the sample-history child list is automatically updated whenever a sample event occurs. A computer-implemented method can detect actions taken by a user with respect to a sample event and record the event in a database. In an alternative embodiment, events can be manually added to the sample-history child list. When events are manually added, a graphical interface is provided to facilitate a user entering the events. While it is advantageous to record a sample history in a child list, any mechanism for tracking events that affect a sample can be used.

Non-limiting examples of sample events include: creating a sample database object, copying a sample database object, copying a library containing a sample, assigning a sample to a plate, changing the status of a plate that contains a sample, changing the status of a library that contains a sample, changing the status of a sample, creating an experiment that is associated with a plate containing a sample, importing results of an experiment conducted on a sample in a laboratory, changing or adding ingredients or processing steps in a sample database object, adding process steps to the post-synthesis treatment child list in a library or plate that contains a sample, and changing the location field of a plate containing a sample.

In one embodiment, the location of a sample at the time of a sample event is also recorded in the sample-history child list for each event. For example, upon the occurrence of a sample event, a computer-implemented method can read the location field for the plate on which the sample is located and record that location with the event in the sample-history child list. In another example, when an experiment is created, the sample event indicating the experiment can also indicate the location of the equipment that is to perform the experiment. This result can be accomplished by a computer-implemented method reading the location field contained within the equipment database object that is referenced in the experiment database object. In still another example, a sample event will be created when the status field in the plate database object is changed to "in transit." The location of the sample recorded at the time of the sample event will indicate that the sample is in transit. The above examples are not limiting and any method for reading and recording the location of a sample in a database at the time of a sample event may be used.

In one embodiment, an interface to laboratory equipment is provided such that sample events are automatically recorded when the equipment takes a particular action. For example, the time that a particular test is conducted on a particular sample in the laboratory can be recorded. Furthermore, if samples are physically moved using automated instrumentation, the location of the samples may be recorded. For example, the sample may be transferred from one plate to another plate or from one well to another well in the same plate. If such transfers are conducted through the use of automated instrumentation, the interface to the instrumentation can provide the exact time of transfer and indicate whether there were any problems in completing the transfer. The information can be used to create a sample event in a database indicating the new location of the sample. Similarly, automated transfer of the location of a plate containing a sample can be reported using the interface.

In another embodiment, the location of a sample is recorded when a physical sample is manually relocated. For example, if a user moves a container that holds a sample from one location to another, an interface can be provided to the user to enter the new location of the container. Alternatively, bar-coding on the sample container can facilitate automatic updating of the database if a sample is manually moved. A user and/or a device can scan ―especially on demand or automatically - the sample container bar-code at the new location. An interface between the bar-code scanner and a database can be provided such that any bar-code scans are detected and the location of the samples in the container is updated in the database.

In another embodiment of the invention, new types of events can be created, e.g. by a user. An interface is provided that allows the user to define which actions will generate an event listing in the sample-history child list. For example, a user may specify that when a particular field in a particular database object is modified, the modification is recorded in the sample-history child list of the sample or samples that are associated with the database object. In one embodiment, the user can also specify what information is recorded in the sample-history child list. For example, the user can specify that the date, time, current location of the sample, or other information is to be recorded with each event.

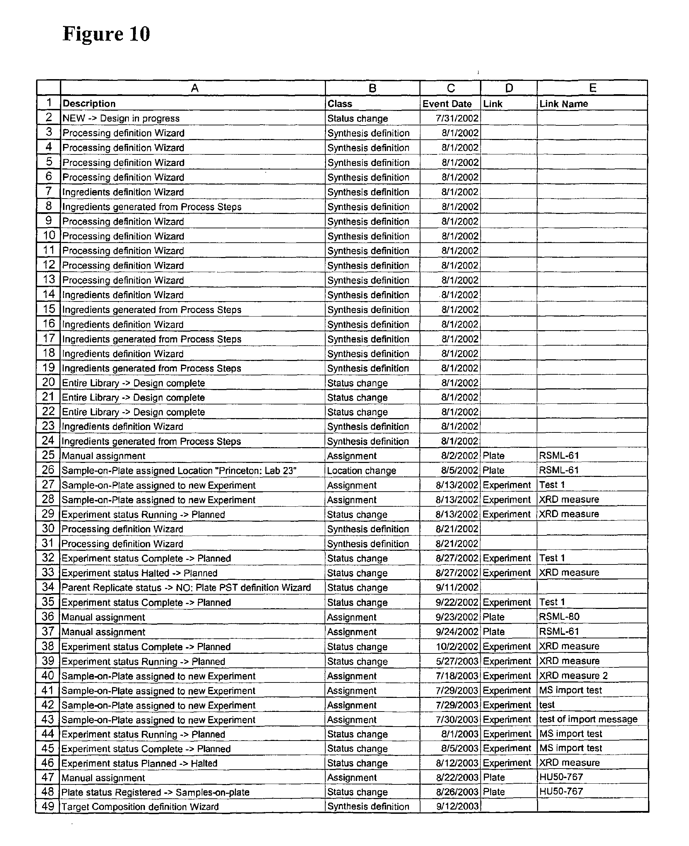

Several means for displaying the history of sample events of a sample can be provided. In one embodiment, a user views the history of sample events by displaying the sample-history child list for the sample. In another embodiment, a graphical interface is provided that enables the user to specify one or more samples. The graphical interface then displays a record of all sample events for the samples. In still another embodiment, after a user specifies one or more samples for which the sample histories are desired, a macro, e.g. an Excel® Visual Basic macro, populates the columns of a spreadsheet with the sample events. A spreadsheet populated with an illustrative sample history is depicted in Figure 10.

In some embodiments, a user can restrict which types of sample events are displayed. For example, a user may only be interested in post-synthesis process steps performed on a sample. In such a case, the user can specify that the only sample events that are to be displayed are process steps added to a sample database object after initial synthesis. Furthermore, a user can specify a range of dates for which sample events are to be displayed. In addition, a user can restrict the display to view only specified elements within the displayed sample events. For example, the user can restrict the display to only indicate the location of the sample for the displayed sample events. In this way, a user can view the current location of a sample by displaying only the location of the sample at the time of the last recorded sample event.

In other embodiments, a user can search a database containing sample database objects using user-specified criteria. The criteria can be based on any information recorded in the database. For example, the user can search for all samples that reside on specified plates or in specified libraries. The user can request that the result of the search be a listing of the complete sample histories for the samples found during the search. Alternatively, the user can restrict the display to certain event types, dates, or event elements as described above. In one embodiment, a macro, e.g. an Excel® macro, populates a column in a database with the current locations of all of the samples returned in a database search.

In one embodiment, users can search a database using sample events as part of the specified search criteria. For example, a user can search for all samples that were located at a particular location on a specified date.

While detailed description has been provided regarding the tracking of sample events, the history of other objects within a FTE database can similarly be tracked. For example, actions that affect an entire library samples can be recorded within library database objects and actions that affect all the samples on a particular plate can be recorded within the plate database object. In another example, equipment can also be recorded. For example, in addition to recording each use of a piece of equipment, any maintenance performed on the equipment can be manually recorded in the equipment database object.

### Automatically Populating the Database with Samples

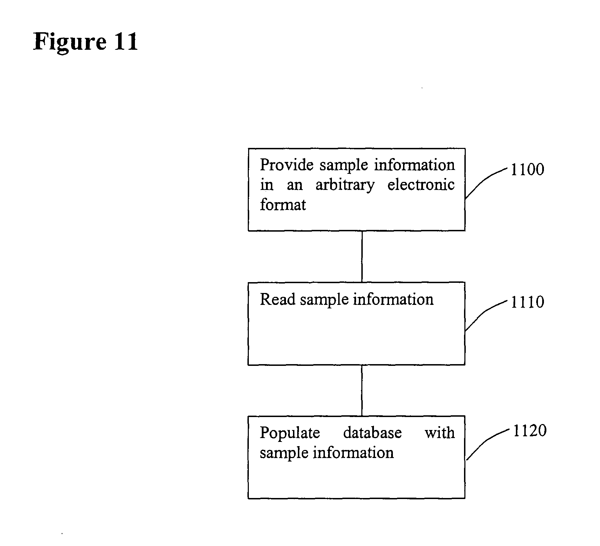

One embodiment of the present invention provides a computer-implemented method to automatically populate a database with information to be included in sample database objects. In this way, sample database objects can be created and/or modified without a user having to enter the information directly into the database interface. In one embodiment, the database is populated from sample information in an arbitrary electronic form. Figure 11 is a flowchart that illustrates this embodiment. First, information to be included in one or more sample database objects is supplied in an arbitrary electronic format (1100). The computer-implemented method then reads the sample information (1110) and populates a database with the information (1120). If the sample database objects already exist, the information is added to the objects. In some cases, information already residing in a sample database object is overwritten. If the sample database object does not already exist, a new sample object is created and is populated with the information supplied in the arbitrary electronic format.

In various embodiments, the arbitrary electronic format (1100) is a text file, a spreadsheet format, output from a library design tool, or output from laboratory instrumentation. However, any electronic format readable by a computer-implemented method may be used.

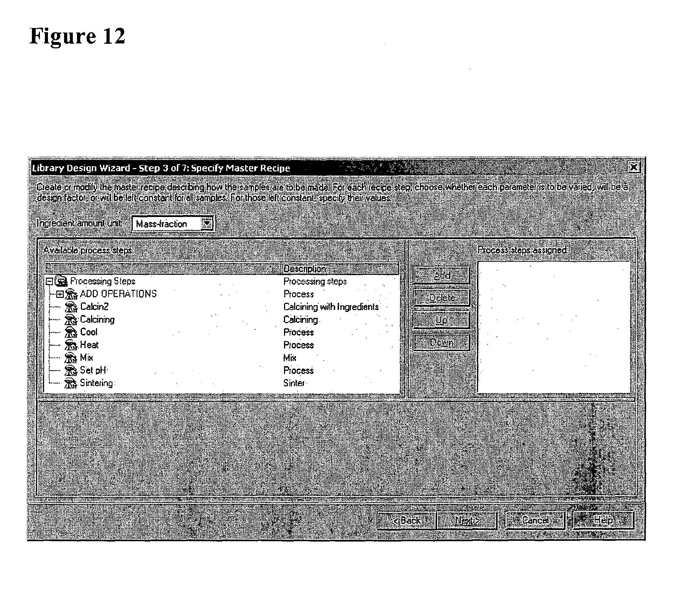

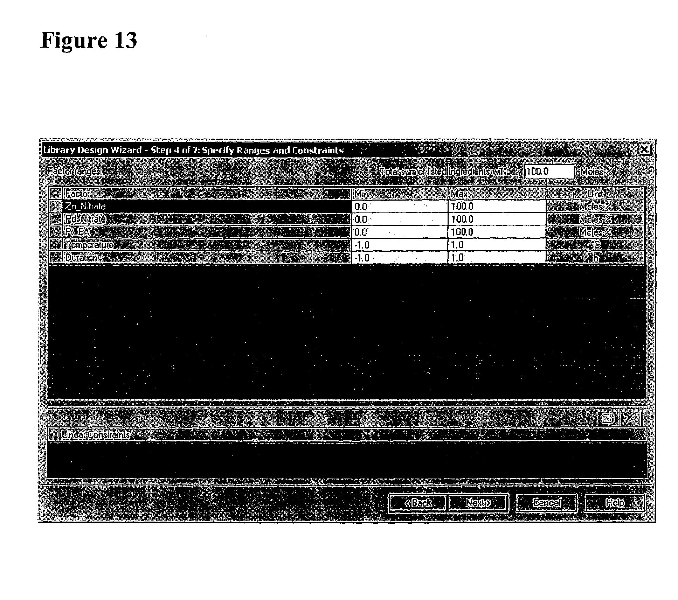

When a library design tool is used, the tool generally takes as input a series of ingredients and processing steps, some of which are allowed to vary over a specified range. The tool then generates a set of sample recipes designed to span the parameter space as broadly as possible using the fewest number of samples. The set of sample recipes can then be read by the computer-implemented method and a database populated with sample database objects. In one embodiment, the library design tool has access to pre-defined database objects such as process steps, ingredients, parameters, and units. In that case, a graphical window can be provided that allows a user to select process steps and specify the parameters that are to be fixed for all samples created by the design tool. An illustrative example of such a window is depicted in Figure 12. For example, a user may select process steps for adding three ingredients, mixing, and heating. The user can specify the ingredients that are to be added, the duration of mixing, and the units for all parameters. However, the user can indicate that the amount of the ingredients to be added and the temperature and duration of heating are to be variable. An additional graphical window can be provided for the user to specify the range over which the amounts of ingredients and the duration of heating are to vary, for example, as depicted in Figure 13. The library design tool can then generate a set of sample definitions that contain parameter values within the specified ranges and the database can be populated with a set of corresponding sample database objects.

In one embodiment, multiple sample definitions are provided in an arbitrary electronic format and a library containing the samples is automatically created in the database. The information provided can contain, among other things, the name of the library to be created, the date that the library is created, the name of each sample, the names of each ingredient to be added to each sample, the amount of each ingredient to be added to each sample, additional processing steps to be performed on each sample (e.g., mixing, heating, etc.), and parameters that specify in more detail the processing steps (e.g., the temperature to be reached in the case of a processing step for heating). Any other information necessary or useful can also be provided.

In another embodiment, sample definitions are provided and the information contained therein is added to a library already existing in the database. The information provided can contain, among other things, the name of the library to which the sample is to be added, the name of each sample, the names of each ingredient to be added to each sample, the amount of each ingredient to be added to each sample, additional processing steps to be performed on each sample (e.g., mixing, heating, etc.), and parameters that specify in more detail the processing steps (e.g., the temperature to be reached in the case of a processing step for heating). Any other information necessary or useful can also be provided.

In one embodiment, when samples are added to the database, the samples are automatically assigned a unique database identifier. In some embodiments, the identifier comprises a number.

In one embodiment, a program written in C++ and Visual Basic reads sample information from a text file and executes SQL commands using an interface to an Oracle® database in order to populate the database with the information. In another embodiment, a program written in Visual Basic and C++ reads electronic output from a library design tool and executes SQL commands using an interface to an Oracle® database in order to populate the database with the information.

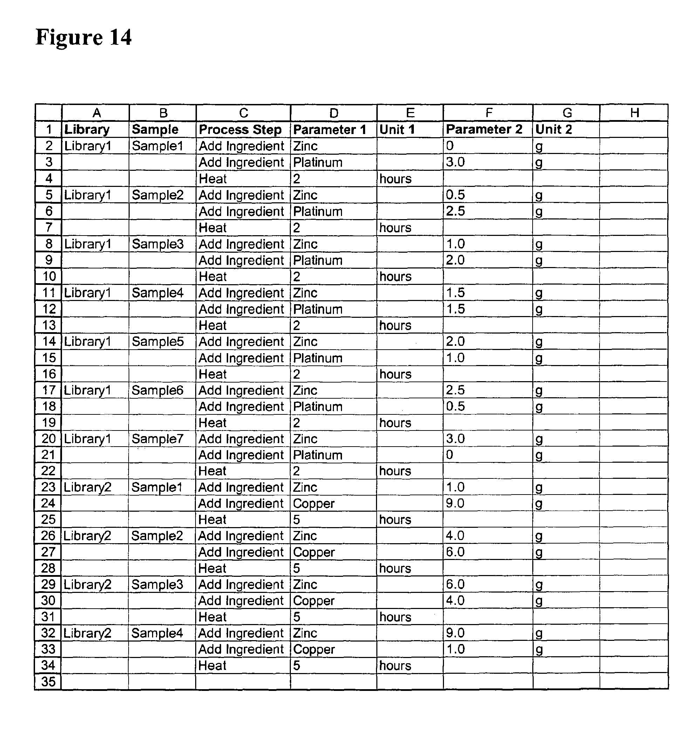

In still another embodiment, a macro, e.g. an Excel® Visual Basic macro, reads sample information from the columns of a spreadsheet, e.g. an Excel® spreadsheet, and populates the database using a specific data object, e.g. an ActiveX Data Object. For example, Figure 14 depicts a spreadsheet containing columns for library name, sample name, process step names, parameters associated with process steps, and units associated with the parameters. This information is read and corresponding sample database objects are populated or created in a database. The spreadsheet depicted in Figure 14 would result in "Library1" being populated with sample database objects named "Sample1", "Sample2", "Sample3", "Sample4", "Sample5", "Sample6", and "Sample7." The process-step child lists for these objects would be automatically populated with the process steps to add two ingredients and heat. The software would reference existing process-step database objects named "Add Ingredient" and "Heat" to determine the parameters that require values and units. These values and units would then be read from the parameter and unit columns in the spreadsheet to complete the population of the process-step child lists. The library database object named "Library2" would similarly be populated with the named samples. In some embodiments, if the named process steps or other named database objects are not already present in the database, they can automatically be created. The example depicted in Figure 14 illustrates the use of 2 parameters and units for the processes steps. However, the method can be applied to an arbitrary number of parameters.

In some embodiments, the invention comprises an Oracle® database and associated application modules in which some of the modules read sample information and populate the Oracle® database with the information. In other embodiments, multiple Oracle® databases are provided such that multiple sites can exchange data and use advanced replication procedures. While it is advantageous to use an Oracle® database, any database or means of storing electronic information may be used.

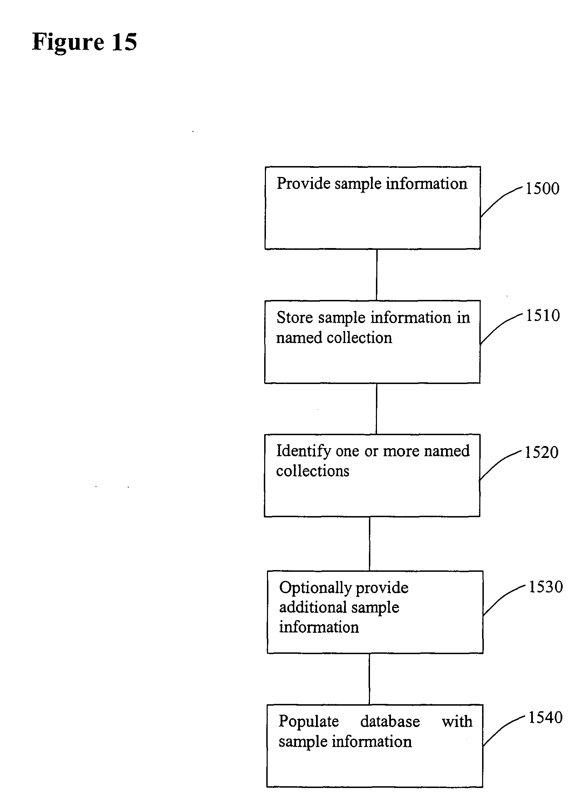

Another embodiment of the present invention is illustrated in the flowchart in Figure 15. As shown in Figure 15, selected sample information is defined (1500) and then stored as a named collection (1510). For example, a series of processing steps can be identified along with ingredients and values for some of the parameters associated with the processing steps. The ingredients and values of other parameters can remain undefined in the collection. After one or more collections are created, sample database objects can be created by identifying one or more collections (1520) and optionally providing the missing ingredients and values along with additional information, such as additional processing steps, for one or more samples (1530). The additional information can be provided by direct user the supplied list. Alternatively, information within in a set of sample database objects may be overwritten with a new constant value. For example, the status field of a set of samples can be overwritten with a "synthesized" status once the samples have synthesized in a laboratory.

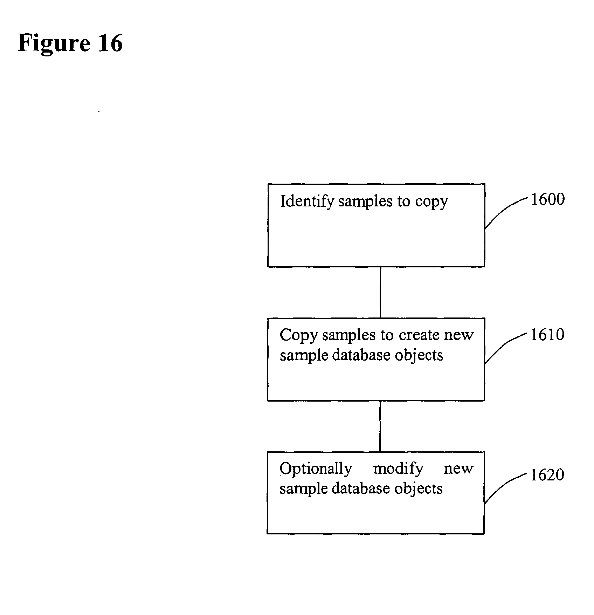

In another embodiment of the invention, depicted in Figure 16, samples already stored in a database can be used as starting points for automatically creating new samples. A list of the sample database objects to be copied can be provided in an arbitrary electronic format along with any modifications or additional information that is to be added. First, the samples to be used as starting points are identified from the provided arbitrary electronic format (1600). The samples can be identified by sample name, unique identifier, the library to which the samples belong, a set of ingredients or process parameters that the desired samples should contain, or some other means. The identified sample database objects are then copied from the database (1610) to create new sample database objects. The new sample database objects can optionally be modified (1620), by appending the new information or by modifying existing information as specified. For example, after FTE has been performed to create a library of samples in the laboratory, it may be desirable to further use these samples as an ingredient to create a new set of samples, such as by adding additional ingredients. Rather than creating the new set of sample database objects from scratch, the original sample database objects can be copied and the new ingredients and processes appended. In this way, less computational and user resources are required to register the new samples in the database. In another example, a desired new set of samples varies only slightly from the original set of samples, such as by replacing one ingredient with another. In this case, it is more efficient to copy the original set of samples and make the desired change to the copies.

In still another embodiment of the invention, sample information provided in an arbitrary electronic format may not be complete enough to create new sample database objects. In that case, a computer-implemented aspect of the invention can provide reasonable default values for the unknown information to the database in order to create the samples. For example, information that is always required for a sample definition may be pre-defined with default information. In some cases, the default information may be a constant. In other cases, the pre-definition includes instructions for how to generate the default information. In another embodiment, a user is prompted to provide the needed information.

While a detailed description for automatically populating a database with sample information has been provided, similar methods may be used to automatically populate a database with any other information. For example, database objects for projects, studies, libraries, process steps, parameters, units, tests, plates, equipment, experiments, and another objects useful for a FTE database may be automatically created and/or modified using the methods described above.

### Importing Experimental Test Results

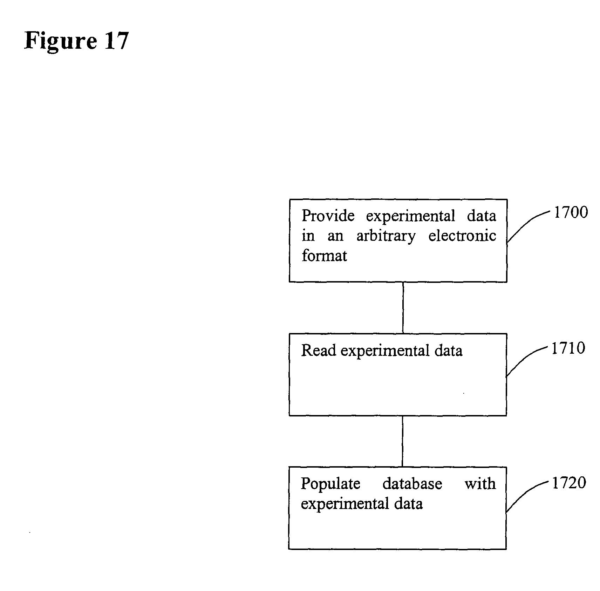

One embodiment of the present invention provides a computer-implemented method to populate a database with the results of experimental measurements. As illustrated in the flowchart in Figure 17, experimental data is provided in an arbitrary electronic format (1700). The computer-implemented method then reads the experimental data (1710) and populates a database with the data (1720).

In various embodiments, the arbitrary electronic format (1700) is direct input from a user, text, a spreadsheet format, or direct output from laboratory instrumentation. However, any electronic format readable by a computer-implemented method can be used. When the arbitrary electronic format comprises direct input from a user, a graphical user interface can be provided to facilitate the user entering the experimental data.

In one embodiment, the experimental data is provided in data files. A user navigates to and selects one or more data files using a graphical interface. The user can navigate to any folder on his computer or to any networked machine that he has permission to view. A SQL procedure reads the file and populates the appropriate tables of the database. This import method can be extended to handle any number of file formats. In a specific embodiment, the graphical interface is based on a Common File Dialog program, e.g. the Windows® Common File Dialog and the interface allows users to select a single file or multiple files. In another specific embodiment, the data files are in a standard format identified as "cmat" format. In an embodiment, a means is provided to translate an arbitrary data file format into the "cmat" format prior to importing into the database.

In one example, prior to importing test results, the experiment database object that is associated with the experiment that produced the results is selected, e.g. by a user. In this way, the experiment-results child list in the experiment database object can be automatically populated with results upon importation of data. The data file that is to be imported can identify one or more tests that the data corresponds to. The identified test database objects can then be referenced to determine the format of the data for each of the identified tests. This information can be used to facilitate reading the experimental data from the data file. Furthermore, when the data is imported, it can be associated with the test that generated the data. In one embodiment, the association is by organizing the experiment-results child list such that the data results for each sample is located under each test conducted in a hierarchical tree view (see Figure 8). In this way, the test parameters that held during the measurement can easily be viewed by selecting the test from the experiment-results child list. While use of the experiment-results child list is advantageous to associate experimental results with the tests that generated them, any means of association can be used.

In one embodiment, the data file is in a "cmat" format. "Cmat" format contains columns of information separated by semi-colons. The initial columns can contain, among other things, the location of the sample on the plate being tested, the date and time of the measurement, and the name of the test that was performed. After the initial identifying columns, the columns can contain the test results. Both raw and scaled data results can be included. If scaled data results are not provided, those columns can be left blank. If the data consists of multiple variables, multiple columns can be used. For example, a data point that is in a triple data format (i.e., x, y, z) is recorded in three successive columns of raw data and three successive columns of scaled data for a total of six columns. If arrays of data are provided, the data column format can be repeated to include all data points. For example, for triple data format, every six columns would constitute one data point (three columns for raw data and three for scaled). Alternatively, instead of providing the data itself in the "cmat" file, a column in the "cmat" file can identify another data file that contains the columns of data. One "cmat" file can contain results from multiple tests applied to multiple samples. While the above-described "cmat" format is advantageous, any file format can be used.

When an experiment-results child list in an experiment database object is populated, the experiment-results child lists in the sample database objects of all samples tested can also automatically be populated. These child lists indicates the tests performed and provides access to view the resulting data. The analytical data can be viewed both in graphical form and a spreadsheet form. A user may also select a test from the experiment-results child list in order to view the information contained within the test database object. Thus, a mechanism is provided for convenient viewing of analytical data for each sample along with the experimental conditions that held when the measurements were performed. Furthermore, by viewing the process step and ingredient child lists in the sample database object, a user can conveniently view all recipe information for the sample. The sample history child list can also be viewed. Thus, the sample database object provides access to all information of interest to a FTE experimenter in one convenient location. While a sample database object as described herein provides one means of associating and accessing all relevant FTE information for a sample, any means to associate sample recipe information, test conditions, experimental results, and sample history can be used.

In one embodiment, the invention provides seamless integration between test definition, test execution, and importation of results in a database. Test instrumentation is interfaced with the database. Once an experiment in the database is defined (i.e., one or more tests to be conducted on a plate of samples), which may be done e.g. by a user, the experiment can be conducted by automated instrumentation. The instrumentation can then import the resulting data directly into the appropriate tables in the database.

### Searching the Database

In one embodiment of the present invention, users an/or a device can construct queries in order to search a FTE database for database objects that satisfy the conditions of the queries. The user can include criteria in the query based on any information contained in the database. For example, a user can search in any of the fields or child lists of the database objects described above, or combinations thereof, for specified information. The criteria can include logic statements using Boolean operators. In addition, when the search criteria includes numerical information, ranges can be provided in which the numerical information is to fall. The user can specify one or more database objects to be returned as a result of the query. Alternatively, the user can specify that only particular pieces of information contained within a database object are to be returned.

In one embodiment, a search can be conducted based on parameters that are part of defined tests. The result of the query can be the experimental results from all experiments conducted that include the tests. In one use, the search criteria can include the names of any parameters without any value specified. For example, a user can search for all experimental results from tests in which "temperature" has been specified as a parameter. In another use, the search criteria can include the names of the parameters along with a specified value. For example, a user can search for all experimental results from tests in which the temperature was greater than 100 degrees Celsius.

In one embodiment, experimental data can be specified in the search criteria. For example, where a particular test measures conductivity, a user could request all samples on which the test had been performed and a certain minimum conductivity had been measured.

In another embodiment, queries can be saved such that they can be used or modified at a later time.

All above-mentioned embodiments may be used as single independent embodiments of the present invention, but may also be combined one with another.

## Claims

1. A method of searching a database, said database comprising a plurality of sample data objects, each sample data object corresponding to a sample that comprises a chemical or composition of chemicals and each sample data object comprising a plurality of data fields populated with sample events, said method comprising:
constructing a query to search for one or more of said sample data objects based on information contained in one or more of said data fields; and
performing a search of said database using said query.

2. The method of claim 1, wherein said data fields include the location of said samples when said sample events occur.

3. The method of claim 2, wherein said location comprises an identification of a plate on which the sample is located.

4. The method of any of claims 1 to 3, wherein said sample events include at least one event selected from the group consisting of creating a sample data object, copying a sample data object, copying a library data object that comprises a reference to a sample data object, assigning a sample data object to a plate data object, changing a status indicator in a plate data object that comprises a reference to a sample data object, changing a status indicator in a library data object that comprises a reference to a sample data object, changing a status indicator in a sample data object, creating an experiment data object that is associated with a plate data object comprising a reference to a sample data object, importing into a computer database results of an experiment conducted on a sample, changing or adding ingredients or processing steps in a sample database object, adding process steps to a library data object or plate data object that comprise references to a sample data object, and changing a location data field in a plate data object that comprises a reference to a sample data object.

5. A method of identifying the location of one or more samples, each sample comprising a chemical or a composition of chemicals, said method comprising:
providing a database comprising sample data objects corresponding to said samples, each of said sample data object comprising a plurality of data fields populated with sample events and locations of said samples when said sample events occur;
retrieving locations from said database corresponding to the sample event recorded last for at least one of said samples; and
populating cells in a spreadsheet with said locations corresponding to the sample event recorded last.

6. The method of claim 5, wherein said retrieving and populating steps are performed by a macro.

7. The method of claim 5 or 6, wherein current locations of all samples stored in said database are identified.

8. The method of any of claims 5 to 7, wherein a user selects which samples stored in said database are identified.

9. The method of any of claims 5 to 8, wherein which samples stored in said database are identified is determined based on criteria identified by a user.

10. The method of claim 9, wherein said criteria does not include a direct identification of said samples.

11. The method of any of claims 5 to 10, wherein a selection is made which samples stored in said database are to be identified by populating cells in said spreadsheet with references to said sample data objects and then executing said macro.

12. A method of populating a database with experimental results obtained from conducting an experiment on a plurality of samples, said method comprising:
providing a database stored on a machine readable medium, the database comprising a plurality of sample data objects, each sample data object corresponding to a sample comprising a chemical or a composition of chemicals;
creating a test data object corresponding to an experiment that can be conducted on said samples;
defining in said test data object information regarding format of data obtained upon conducting said experiment;
selecting a data file using a Common File Dialog program, said data file comprising experimental results obtained from conducting said experiment on said samples; and
using said format, reading said data file and populating data fields with said experimental results for each said sample.

13. The method of claim 12, wherein said information includes at least one data parameter, each said data parameter indicating a type of laboratory variable, or at least one data unit.

14. The method of claim 12 or 13, further comprising converting said data file from one data format to another data format that comprises columns of data corresponding to said parameters.

15. The method of any of claims 12 to 14, wherein multiple data files are selected and read during the selecting and reading steps.

16. A graphical user interface having means to show experimental data populated according to the method of any of claims 12 to 15.

17. A method of populating a database with information pertaining to a plurality of samples, each sample comprising a chemical or composition of chemicals, the method comprising:
providing in an arbitrary electronic format information corresponding to ingredients and/or process steps used to synthesize each said sample;
reading said information in arbitrary electronic format; and
populating data fields in said database with said ingredients and process steps for each said sample.

18. The method of claim 17, wherein said arbitrary electronic format is a text file or a spreadsheet.

19. The method of claim 17 or 18, wherein said reading step comprises executing a macro.

20. The method of claim 18 or 19, wherein said spreadsheet comprises cells populated with identifiers of each said sample and a cell populated with an identifier of at least one library data object, the method further comprising populating data fields in said library data object with references to said samples.

21. The method of claim 18, wherein said arbitrary electronic format is output from a library design tool.

22. The method of any of claims 17 to 21, wherein said populating step comprises creating a sample data object corresponding to each said sample and populating data fields in said sample data object with said ingredients and process steps.

23. The method of any of claims 17 to 22, further comprising creating a library data object and populating data fields in said library data object with references to said sample data objects.

24. The method of claim 23, further comprising populating data fields in a pre-existing library data object with references to said sample data objects.

25. The method of any of claims 17 to 24, wherein said populating step comprises populating data fields in pre-existing sample data objects corresponding to each said sample with said ingredients and process steps.

26. The method of claim 25, wherein said pre-existing sample data object comprises data fields populated with some ingredients and/or process steps prior to said populating step.

27. The method of claim 14 or 17, wherein said step of reading a data file and populating data fields or said populating step comprises executing a SQL command.

28. The method of any of claims 17 to 27, further comprising providing in said arbitrary electronic format a reference to a named collection stored on a machine readable medium, said named collection comprising additional ingredients and/or process steps used to synthesize said samples, where said reading and populating steps further comprise reading said named collection and populating said data fields with said additional ingredients and process steps.

29. The method of any of claims 17 to 28, further comprising copying sample data objects in said database to create new sample data objects, said sample data objects comprising data fields populated with additional ingredient and process step information, wherein said populating step comprises populating additional data fields in said new sample data objects with said ingredient and process step information provided in said providing step.

30. The method of claim 17 to 28, further comprising copying sample data objects in said database to create new sample data objects, said sample data objects comprising data fields populated with additional ingredient and process step information, wherein said populating step comprises overwriting at least some of said data fields in said new sample data objects with said ingredient and process step information provided in said providing step.

31. The method of claim 29 or 30, wherein said sample data objects to be copied are identified in said arbitrary electronic format.

32. A graphical user interface adapted to receive an input data and use said input data to construct a query according to the method of claim 1 or to identify samples and adapted to determine the location of said samples using the method of claim 5 or to perform the selecting step in the method of any of claims 12 to 15 or to provide said information in the method of claim 17 and said interface adapted to perform said reading and populating steps according to the method of claim 17.

33. The graphical user interface of claim 32, wherein said input signal is a name of a data file.

34. The graphical user interface of claim 32 or 33, wherein said arbitrary electronic format is a specific spreadsheet and said input executes a macro.
